# EUROPEAN PATENT APPLICATION

(11) **EP 4 066 795 A1**
(43) Date of publication of application: **05.10.2022**
(21) Application number: 20892366.4
(22) Date of filing: 31.08.2020
(51) Int. Cl.: A61F 2/966, A61B 17/221

(54) **DELIVERY GUIDE WIRE AND THERAPEUTIC DEVICE**

(30) Priority: 27.11.2019 CN 201911183832
(71) Applicant: MicroPort NeuroTech (Shanghai) Co., Ltd., Shanghai 201318 (CN)
(72) Inventor: LONG, Ping, Shanghai 201318 (CN); TIAN, Hao, Shanghai 201318 (CN); HOU, Juan, Shanghai 201318 (CN)
(74) Representative: Patentship Patentanwaltsgesellschaft mbH
(86) International application number: PCT/CN2020/112448
(87) International publication number: WO 2021/103720

(57) **Abstract**

A delivery guide wire (10,100) and a therapeutic device are disclosed. The therapeutic device includes the delivery guide wire (10, 100), a medical implant and a delivery catheter (20). The delivery guide wire (10, 100) includes a core shaft (110) and a driving member (120) disposed on the core shaft (110), and the driving member (120) defines thereon a depression. The medical implant is compressed by the delivery catheter (20) and disposed over the delivery guide wire (10,100) in such a manner that it is at least partially received in the depression. This results in an increased contact area between the medical implant and the delivery guide wire (10, 100), which facilitates movement of the medical implant in sync with the delivery guide wire (10, 100) and makes its delivery easier.

## Description

### TECHNICAL FIELD

The present invention relates to the technical field of medical instruments and, more specifically, to a delivery guide wire and a therapeutic device.

### BACKGROUND

Most intracranial aneurysms are visualized as abnormal ballooning in the walls of cerebral arteries and are the No. 1 cause of subarachnoid hemorrhage. Among cerebrovascular diseases, with incidence being second only to that of cerebral thrombosis and hypertensive cerebral hemorrhage, intracranial aneurysms are extremely risky and dangerous.

Currently, there are essentially three options for treating intracranial aneurysms: 1) surgical clipping, involving blocking cerebral blood circulation to the aneurysm by clipping the base thereof with a metal clip, thus not only restoring the parent artery's normal blood supply but also preventing its rupture and consequential bleeding; 2) intra-aneurysmal embolization, involving placing a embolizing material in the aneurysm for embolization thereof, which can prevent further expansion of the aneurysm that may ultimately lead to rupture and consequential bleeding; and 3) endovascular stenting, involving implanting a stent into the artery to reduce blood flow therein to the aneurysm, thus causing blood stagnation and thrombus formation in the aneurysm, which facilitates the closure of the aneurysm and lowers the risk of rupture. As aneurysms typically occur around the circle of Willis where there are many important blood vessels, nerves and brain tissues, the surgical clipping of an aneurysm is very challenging to the operating physician, and the patient mortality rate has been found to reach as high as 50%. For complex aneurysms, such as large and giant ones, simple reliance on intra-aneurysmal embolization is problematic because frequent recurrence has been confirmed. For these reasons, endovascular stenting is most commonly chosen nowadays for the treatment of intracranial aneurysms.

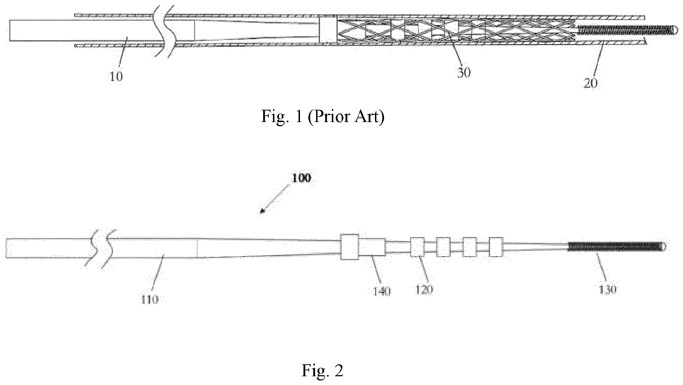

An existing therapeutic device for endovascular stenting of an intracranial aneurysm is shown in Fig. 1. The therapeutic device includes a delivery guide wire 10 and a delivery catheter 20. The delivery catheter 20 defines a lumen, which axially extends through the catheter, and in which a stent 30 to be delivered is accommodated so as to be disposed over the delivery guide wire 10 by interference fits between the delivery guide wire 10, the stent 30 and the delivery catheter 20. In this way, when an operator is advancing the delivery guide wire 10 within the delivery catheter 30, a first friction force generated between the delivery guide wire 10 and the stent 20 will urge the stent 30 to move in sync with the delivery guide wire 10 until it reaches a predetermined site. In this process, a second friction force opposite to the first friction force will be created between the stent 30 and an inner wall of the delivery catheter 20, so as to resist the stent 30 from moving with the delivery guide wire 10. Since the endovascular stent provides a therapeutic effect to the intracranial aneurysm by guiding the flow of blood, it is desired to have high metal coverage, and since intracranial blood vessels are typically thin and tortuous, it requires to be delivered using a very small and thin delivery device with desirable compliance. This means that a larger size of the delivery device will more tightly fit against the stent, making the second friction force created during delivery too significant to allow easy advancement of the stent.

In order to enable easy advancement within the delivery catheter 30, the existing delivery guide wire 10 typically has a smooth outer surface, and in order to increase the first friction force between the delivery guide wire 10 and the stent 30, driving members with a relatively high coefficient of friction is sometimes provided on the delivery guide wire 10. However, the existence of the driving members will lead to a larger outer diameter of the stent on the delivery guide wire 10, increasing the second friction force between the stent and the inner wall of the delivery catheter 30. Additionally, the entire therapeutic device may consequently have an expanded outer diameter, making it less suitable for use in the treatment of distant diseased blood vessels. Further, a larger number or a greater total length of such driving members may lead to reduced distal compliance of the delivery guide wire 10.

### SUMMARY OF THE INVENTION

It is an object of the present invention to provide a delivery guide wire and a therapeutic device, with increased ease of stent delivery.

In pursuit of this aim, the present invention provides a delivery guide wire for use for delivering a medical implant, which comprises a core shaft and a driving member on the core shaft, the driving member defining thereon a depression.

Optionally, the driving member may comprise a body and a recess formed in an outer surface of the body, the recess forms the depression.

Optionally, a structure of the recess may match at least part of a structure of the medical implant when the latter is in a crimped configuration.

Optionally, the recess may be structurally mirrored to an inner surface of the medical implant in the crimped configuration.

Optionally, a width of the recess may range from 0.0008 inches to 0.004 inches. Additionally or alternatively, a depth of the recess may range from 0.0002 inches to 0.004 inches.

Optionally, the recess may comprise one recessed element or a plurality of recessed elements which are interlaced or continuous with, or spaced apart from, one other across the outer surface of the body.

Optionally, the recess may helically extend in the outer surface of the body about an axis thereof to form one or more helical grooves.

Optionally, the driving member may be composed of a wound wire which helically extends about an axis of the core shaft to form one or more helices, with adjacent turns spaced apart to form the depression.

Optionally, the wound wire may be a polymeric wire or a metal wire coated on its surface with a polymeric coating.

Optionally, the metal wire may be visualizable. Additionally or alternatively, the metal wire may be a platinum-tungsten alloy wire or a platinum-iridium alloy wire.

Optionally, the driving member may comprise an inner piece and an outer piece, the inner piece made of a metallic material and fixedly sleeved over the core shaft, the outer piece made of a polymeric material and fixedly sleeved over the inner piece.

Optionally, the inner piece may define an interspace, which is partially or entirely filled by the outer piece, wherein at least part of the outer piece goes through the interspace to connect to the core shaft.

Optionally, the inner piece may have an outer surface provided therein with a recess which forms the interspace.

Optionally, the inner piece may comprise a plurality of coils which are arranged along an axis of the core shaft, and the interspace is formed between adjacent coils.

Optionally, the inner piece may be a meshed tubular structure braided from a wire, and openings in the meshed tubular structure fom the interspace.

Optionally, the wire may have a diameter of 0.001 inch or less. Additionally or alternatively, each inch of the inner piece may contain 15-50 braid knots.

Optionally, the inner piece may inlcude at least one tubular element and is partially or entirely wrapped by the outer piece.

Optionally, the inner piece may be a helix formed by a wire extending helically about an axis of the core shaft, with the interspace being formed between adjacent turns of the wire.

Optionally, the wire may have a diameter of 0.001 inch or less. Additionally or alternatively, the helix formed by the wire may have a pitch of 0.001-0.007 inches.

Optionally, the inner piece may be made of a visualizable metallic material selected from one or more of platinum, gold, tungsten, a platinum-gold alloy, a platinum-tungsten alloy, a platinum-iridium alloy and a platinum-nickel alloy.

Optionally, the inner piece may be welded or glued to the core shaft. Additionally or alternatively, the outer piece may wrap the inner piece and extend so as to connect to the core shaft.

Optionally, the outer piece may be made of a material comprising one or more of a block polyether amide resin, a thermoplastic polyurethane elastomer, silicone, nylon and an acrylic polymer.

Optionally, the outer piece may be formed on the inner piece by hot pressing and/or dipping. Alternatively, the inner piece may be adhesively bonded to the outer piece.

Optionally, at least two of the driving members may be provided on the core shaft so as to be spaced apart along the axis of the core shaft.

Optionally, adjacent driving members may be spaced apart by a distance ranging from 0.5 mm to 150 mm.

Optionally, adjacent driving members may be spaced apart by a distance ranging from 0.5 mm to 5 mm.

Optionally, one or more of the driving members may be provided on the core shaft each having an outer diameter of 0.01 -0.03 inches and a length of 0.5-8 mm.

Optionally, the length of each driving member may range from 0.5 mm to 4 mm.

Optionally, the delivery guide wire may further comprise a first visualization member and a second visualization member, the first visualization member disposed at a distal end of the core shaft, the second visualization member disposed on the core shaft, wherein the driving member is situated between the first visualization member and the second visualization member.

In pursuit of the above aim, the present invention also provides a therapeutic device comprising a delivery catheter, a medical implant and the delivery guide wire as defined above, the delivery catheter defining a lumen extending therethrough axially, the lumen configured to receive the medical implant therein in such a manner that the medical implant is compressed against a wall of the lumen and thus assumes a crimped configuration where it is disposed over the driving member so as to be at least partially received in the depression.

Optionally, the lumen may have a radial size ranging from 0.017 inches to 0.029 inches.

Optionally, the medical implant may be a self-expanding stent.

The delivery guide wire and the therapeutic device of the present invention have the following advantages over the prior art:
The therapeutic device includes a delivery guide wire, a medical implant and a delivery catheter, and the delivery guide wire includes a core shaft and a driving member which is disposed on the core shaft and defines a depression. During delivery of the medical implant by the delivery guide wire, the medical implant is received in the delivery catheter in such a manner that it is compressed against a wall of a lumen of the delivery catheter and thus assumes a crimped configuration where it is disposed over the driving member so as to be at least partially received in the depression. This design allows an increased contact area between the stent and the driving member, which results in greater friction between the medical implant and the delivery guide wire, as well as in increased neatness and smoothness of a portion of the medical implant where it is brought into contact with the delivery catheter and hence reduced friction between the medical implant and the delivery catheter and easier delivery of the medical implant. Moreover, the medical implant crimped in the delivery catheter will have a reduced outer diameter, which in turn allows the delivery catheter to have a reduced outer diameter. As such, the therapeutic device can reach a more distant target lesion site and thus have a wider scope of indications. Further, the therapeutic device has enhanced overall compliance which enables it to pass through tortuous blood vessels, resulting in a higher surgical success rate.

Additionally, in some embodiments, the driving member includes an inner piece which is made of a metallic material and disposed over the core shaft and an outer piece which is made of a polymeric material and disposed over the inner piece. Since the inner piece is fixed (e.g., welded) to the core shaft and both of them are made of metallic materials, they strongly attach to each other without displacement therebetween. In addition, the fixed attachment of the polymeric outer piece to the inner piece is accomplished by a special structural design. For example, an interspace may be defined in the inner piece, which is entirely or partially filled by the outer piece. In this way, an intermeshing fit can be achieved between the inner and outer pieces. As another example, the outer piece may wrap the inner piece while extending over and thus connecting to the core shaft so that the outer piece, the inner piece and the core shaft are tightly attached together. Arranging the outer piece over the core shaft via the inner piece that has a relatively large contact area with the outer piece allows increased attachment strength. Compared with conventional connection, these designs of the present invention all enable firm attachment of the entire driving member to the core shaft, thus avoiding loosening, wrinkling or displacement of the driving member during delivery of the stent. As a result, improved reliability of the delivery guide wire during delivery of the medical implant is achieved.

### BRIEF DESCRIPTION OF THE DRAWINGS

Fig. 1 is a schematic diagram showing the structure of an existing therapeutic device;
Fig. 2 is a schematic diagram showing the structure of a delivery guide wire according to an embodiment of the present invention, in which a depression is not shown;
Fig. 3 is a schematic diagram showing the structure of the delivery guide wire according to an embodiment of the present invention, in which the depression is shown;
Fig. 4 is a schematic diagram showing the structure of a stent according to an embodiment of the present invention;
Fig. 5 schematically illustrates a variant of the delivery guide wire of Fig. 3;
Fig. 6 schematically illustrates another variant of the delivery guide wire of Fig. 3;
Fig. 7 is an enlarged schematic view of portion A of the delivery guide wire of Fig. 6;
Fig. 8 schematically illustrates yet another variant of the delivery guide wire of Fig. 3;
Fig. 9 is an enlarged schematic view of portion B of the delivery guide wire of Fig. 8;
Fig. 10 schematically illustrates a further variant of the delivery guide wire of Fig. 3;
Fig. 11 is a schematic diagram showing the structure of a delivery guide wire according to another embodiment of the present invention;
Fig. 12 is a schematic diagram showing the structure of a delivery guide wire according to yet another embodiment of the present invention; and
Fig. 13 is a schematic diagram showing the structure of a delivery guide wire according to still yet another embodiment of the present invention.

In these figures,
10 and 100 denote delivery guide wires;
110, a core shaft;
120, a driving member; 121, a body; 122, a recess; 123, a gap; 124, an inner piece; 125, an outer piece, 126, an interspace;
130, a first visualization member;
140, a second visualization member;
20, a delivery catheter;
30 and 300, stents;
310, filaments; and
311, braid knots.

### DETAILED DESCRIPTION

Objects, advantages and features of the present invention will become more apparent from the following more detailed description thereof made in conjunction with the accompanying drawings. Note that the figures are provided in a very simplified form not necessarily drawn to exact scale for the only purpose of helping to explain the disclosed embodiments in a more convenient and clearer way.

As used herein, the singular forms "a", "an" and "the" include plural referents and the term "plurality" means two or more, unless the context clearly dictates otherwise. As used herein, the term "or" is generally employed in the sense of "and/or", unless the context clearly dictates otherwise. The terms "mounting", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two components. Those of ordinary skill in the art can understand the specific meanings of the above-mentioned terms herein, depending on their context. Like numerals indicate like elements throughout the accompanying drawings.

As used herein, the terms "proximal" and "distal" are employed to describe relative orientations, relative positions and directions between components of a medical device or actions thereof, as viewed by one operating the device. Without wishing to be limiting, a "proximal end" usually refers to an end closer to the operator, and a "distal end" usually refers to an end that enters a patient first, during normal operation of the medical device.

The present embodiment provides a delivery guide wire used to deliver a medical implant to a predetermined site in the body of a patient. The medical implant is, for example, a self-expanding (or self-expandable) stent. The self-expanding stent may be particularly a braided or cut stent. In other embodiments, the medical implant may be instead a medical embolization coil, a blood vessel occlusion device or the like, without limiting the present invention in any sense. In the following, for ease of description, the medical implant is described as being implemented as a self-expanding stent, as an example. For the sake of brevity, the self-expanding stent is referred to as the "stent" hereinafter.

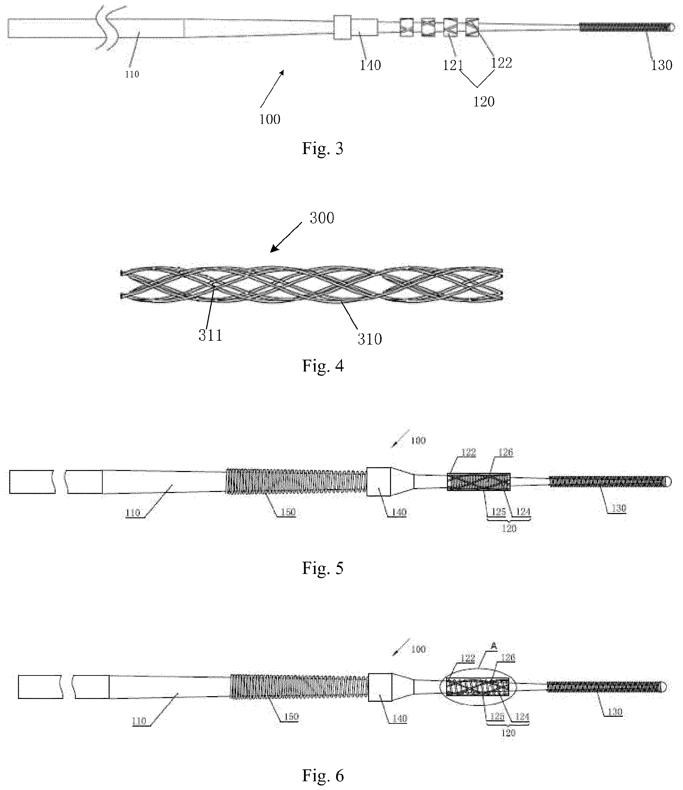

Referring to Figs. 2 and 3, the delivery guide wire 100 includes a core shaft 110 and a driving member 120 on the core shaft 110, and a depression is defined in the driving member 120.

While being delivered, the stent is crimped within a lumen of a delivery catheter (in this configuration, the stent is being contracted) so as to be tightly disposed over the driving member 120 so that the stent radially compresses the driving member 120 and is at least partially received in the depression. This results in a significantly increased contact area between the stent and the driving member 120. When the operator pushes the delivery guide wire 100 to axially advance it in the delivery catheter, a significantly increased first friction force can also be created between the stent and the driving member 120, which can facilitate the movement of the stent in sync with the delivery guide wire 100. When the stent is fabricated by braiding, it may be braided from filaments with different thicknesses. Similarly, when the stent is fabricated by cutting, it may be composed of struts with different thicknesses. In these cases, compressed by the delivery catheter, thicker filaments or struts of the stent would be more forced into the depression, leaving a neater, smoother surface of the stent where it is brought into contact with the delivery catheter (i.e., an outer surface of the stent), which can reduce a second friction force created between the stent and an inner wall of the delivery catheter and hence resistance to advancement of the pushed stent.

As with traditional delivery guide wires, the delivery guide wire 100 of the present embodiment further includes a first visualization member 130 and a second visualization member 140. The core shaft 110 has a first distal end and an opposing first proximal end. The first visualization member 130 may be a visualization coil disposed at the first distal end, and the second visualization member 140 may be arranged on the core shaft 110, with the driving members 120 and thus the stent being positioned between the first visualization member 130 and the second visualization member 140.

In some embodiments, the driving member 120 may be formed of a polymeric material such as silicone, a thermoplastic polyurethane (TPU) elastomer, polyimide, a thermoplastic elastomer (Pebax), polytetrafluoroethylene (PTFE) or the like. In some other embodiments, the driving member 120 may be formed of a metallic material such as stainless steel, a nickel-titanium alloy, a platinum-tungsten alloy or the like.

A number of, e.g., one, two, three, four, five, six or even more of the above-described driving members 120 may be provided on the core shaft 110, and the number of such driving members 120 may depend on a length (i.e., axial dimension) of the stent to be delivered. In general, if each driving member 120 has a fixed length, then the longer the stent is, the greater the resistance it will encounter during delivery, requiring more driving members 120 to be included to provide a greater first friction force between the delivery guide wire 100 and the stent to ensure movement of the stent in sync with the delivery guide wire 100. In this embodiment, each driving member 120 has an outer diameter between 0.01 inch and 0.03 inches and a length between 0.5 mm and 8 mm, preferably between 0.5 mm and 4 mm. When two or more driving members 120 are provided on the core shaft 110, they may be space apart from each other along an axis of the core shaft 110. Optionally, adjacent driving members 120 may be spaced at a distance between 0.5 mm and 150 mm, preferably between 0.5 mm and 5 mm.

It is easier to obtain a great first friction force by properly designing the number of those driving members 120 on the core shaft 110 as well as their size, compared to simply increasing their own coefficient of friction. Moreover, compared to providing one continuous, relatively long driving member on the core shaft 110, a reduced total driving member length and hence higher retrievability of the stent can be achieved (i.e., the shorter the total driving member length is, the more retrievable the stent will be) by providing at least two shorter spaced driving members 120 on the core shaft 110. In addition, by providing a plurality of shorter spaced driving members 120, the delivery guide wire 100 can have improved compliance, allowing the delivery guide wire 100 to more easily pass through tortuous blood vessels. Further, in the cases of the plurality of shorter driving members 120 being included, these driving members 120 can be made of a relatively hard material (with a low coefficient of friction), which can facilitate tolerance control and reduce fabrication complexity. It would be appreciated that the retrievability of a stent can be calculated according to: Stent Retrievability = (Total Stent Length - Distance from Proximal Stent End to Driving members)/Total Stent Length ^{∗}100%, as commonly known to those skilled in the art.

The structure of the driving member 120 will be explained below with reference to the accompanying drawings. It is to be understood that the specific structures of the driving member 120 described in the following embodiments are merely optional implementations of the present invention and not intended to limit the invention in any sense.

Referring to Figs. 2 to 4, in one embodiment, the driving member 120 includes a body 121 which appears like a hollow tube and is disposed over the core shaft 110. A recess 122, i.e., the aforementioned depression, is formed in an outer surface of the body 121. It is to be understood that, in the present embodiment, the "recess" may take any of various forms as practically required, including an elongate groove and circular, square or irregular concave cavities (i.e., pits).

For example, with continued reference to Fig. 3, the structure of the recess 122 at least partially matches the structure of the stent that is being crimped. In particular, the matching may be done in terms of shape and position. Moreover, the size of the recess 122 may be compatible with that of the struts or filaments in the stent. For example, a width of the recess 122 is greater than, equal to or slightly less than a width of the filaments or struts, and a depth of the recess 122 (the distance from its lowest point to its top edges at the outer surface of the body 121) is greater than, equal to or less than a radial size of the filaments or struts. Depending on the size of the stent, the width of the recess 122 may range from 0.0008 inches to 0.004 inches, and the depth thereof from 0.0002 inches to 0.004 inches. It would be appreciated that the width of the recess 122 is designed in correspondence with the width of the stent's filaments or struts. For example, if the filaments extend circumferentially, then their width refers to the size of them measured along an axis of the stent. The width of the recess 122 refers to its size measured along the axis of the stent.

The recess 122 may either be formed as a continuous groove in the outer surface of the body 121, or include a plurality of recessed elements which may be interlaced or continuous with, or spaced apart from, one other.

Fig. 4 shows an example of the stent 300 braided from multiple filaments 310. For this stent 300, the recess 122 may include a plurality of recessed elements which cross one other so that the recess 122 is complementary in shape to an inner surface of the stent when the latter is in a crimped configuration (as shown in Fig. 3). That is, the recess 122 is sized and arranged in the same manner in which the filaments 310 in the crimped stent are sized and arranged. When the stent 300 is disposed over the delivery guide wire 100, each filament 310 may be at least partially received in the depression.

In this embodiment, the recess 122 may be formed in various ways. The body 121 may be formed from a polymeric material, and the formation may involve helically or crossedly winding a wire around the body 121 about an axis thereof when it has been molded but not solidified yet so that the wire is brought into close contact with a surface of, and radially compresses, the body 121. Under the action of this, the outer surface of the body 121 may be inwardly deformed toward the axis of the body 121 at the portions in contact with the wire, resulting in the formation of the recess 122. After that, the wire is removed. The wire made be a metallic, polymeric or other wire. In this embodiment, a diameter of the wire is the same as that of the filaments in the stent 300 to be disposed over the delivery guide wire 100. In other embodiments, the diameter of the wire may be slightly greater or smaller than that of the stent's filaments. Alternatively, the recess 122 may be formed after the body 121 has solidified. For example, it may be heated and softened and then patterned with a wire to form the recess 122. Alternatively, the recess 122 may be engraved in the outer surface of the body 121 that has solidified.

When the body 121 is made of a metal, the recess 122 may also be engraved (e.g., laser-engraved) in the outer surface of the body 121.

When the stent is a cut stent, the recess 122 complementary to the stent may also be formed in the surface of the body 121 by engraving.

Referring to Figs. 5 to 10, in some embodiments, the driving member 120 includes an inner piece 124 and an outer piece 125. The inner piece 124 is made of a metallic material and fixedly disposed over the core shaft 110, while the outer piece 125 is made of a polymeric material and fixedly disposed over the inner piece 124.

The inner piece 124 may be formed of metal, and adhesively bonded, welded or otherwise 10 attached to the core shaft 110 with attachment strength that is high enough to disallow displacement of the inner piece 124 on the core shaft 110. The outer piece 125 is connected to the core shaft 110 by the inner piece 124. In this way, an increased attachment area of the outer piece 125 with the core shaft 110 is achieved, which results in enhanced attachment of the outer piece 125 to the core shaft 110 and a reduced risk of loosening, wrinkling or displacement of the outer piece 125 during delivery of the stent. It would be appreciated that, as used herein, the phrase "disposed over" means either that the inner piece 124 is separate from the core shaft 110 and assembled with the same after they are fabricated, or that the inner piece 125 and the core shaft 110 are integrally fabricated so that the inner piece 124 extends outwardly from an outer surface of the core shaft 110.

Additionally, an interspace 126 may be formed on the inner piece 124 and totally or partially filled by the outer piece 125, in order to result in an increased attachment area between the outer piece 125 and the inner piece 124.

According to embodiments of the present invention, the inner piece 124 may assume any of multiple forms, and some preferred ones are explained below with reference to the annexed figures. It is to be understood that the various forms of the inner piece 124 described below are only optional implementations of the present invention and should not be taken to limit the invention in any sense.

As shown in Fig. 5, in one embodiment, the inner piece 124 may consist of a plurality of coils sleeved over the core shaft 110, which are arranged side by side along the axis of the core shaft 110. In this embodiment, the coils may be formed from a metal wire with a circular or elliptical cross section. Therefore, when adjacent coils are brought into contact with each other, quasi V-shaped recesses, i.e., the interspace 126, may be formed therebetween. In other embodiments, depending on the cross-sectional shape of the coils, the recesses may alternatively be U-like grooves, or cubic, rectangular parallelepiped or hemispherical pits. The outer piece 125 may be formed over outer surfaces of the coils, and the formation may involve hot pressing and/or dipping, molding and shaping, and cooling. The hot pressing approach may include disposing a polymer tube over the inner piece 124 and a heat shrink tube over the polymer tube. After that, the heat shrink tube may be heated and shaped in a mold so that the melted material of the polymer tube penetrates into the interspace 126 in the inner piece 124. After the polymeric material cools and cures, the heat shrink tube may be removed. Examples of the material of the outer piece 125 may include any of a thermoplastic elastomer such as a block polyether amide (Pebax) resin or a thermoplastic polyurethane (TPU) elastomer, silicone, nylon, an acrylic polymer or another polymeric material, or combinations thereof. Filling the polymeric material in the interspace 126 allows a larger contact area and thus stronger attachment between the outer piece 125 and the inner piece 124. Before the polymeric material cures, it may even flow up to the surface of the core shaft 110 between adjacent coils and/or beyond both ends of the inner piece 124, resulting in more tight adhesive attachment between the outer piece 125, the inner piece 124 and the core shaft 110, and thus resulting in an additional reduction in the risk 10 of wrinkling, loosening or displacement of the outer piece 125. In alternative embodiments, the outer piece 125 may be formed first, and then attached to the inner piece 124 for example, by gluing.

In some embodiments, the multiple coils may be spaced over the core shaft with the interspace being formed between adjacent coils. In this case, every pair of the outer piece, the inner piece and the core shaft may be adhesively bonded together.

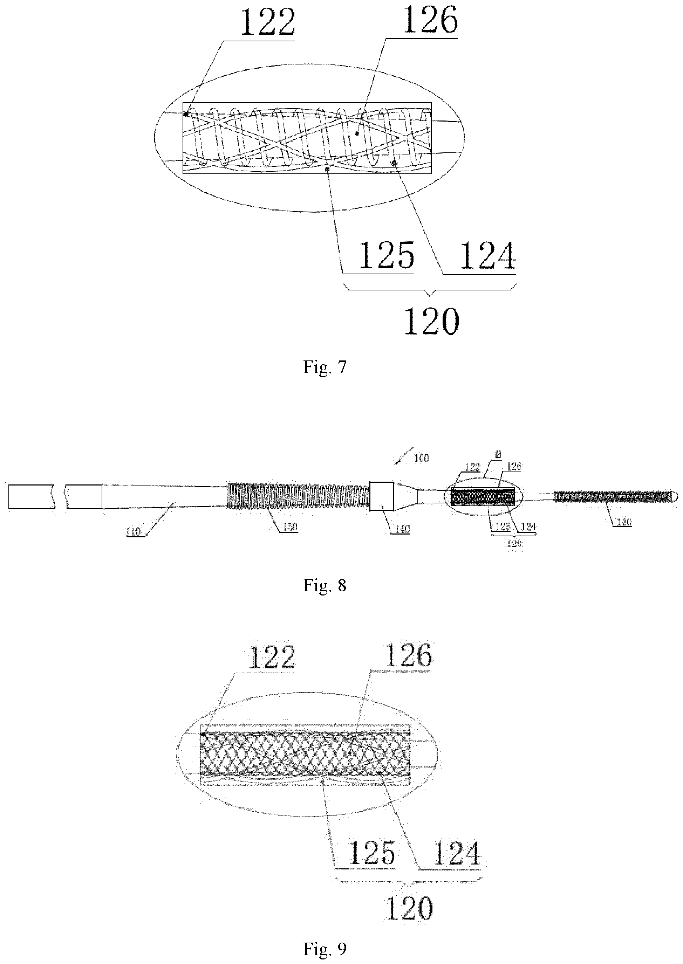

Referring to Figs. 6 and 7, in another embodiment of the present invention, the inner piece 124 is a helical structure formed by helically winding a wire on the core shaft 110 along the axis thereof. Similar to the previous embodiment, adjacent turns of this helical inner piece 124 may be brought into contact with, or spaced apart from, each other. In the present embodiment, one or more of the above-described driving members 120 may be provided on the core shaft 110.

Optionally, in the present embodiment, the wire from which the inner piece 124 is formed may be a polymeric or metal wire. In the latter case, the resulting helical structure may be flexible and easily bendable, making the delivery guide wire 100 desirably compliant as a whole.

Optionally, in the present embodiment, a diameter of the wire from which the inner piece 124 is formed may be less than or equal to 0.001 inch, and the helical structure of the inner piece 124 formed by the wire may have a pitch of 0.001-0.007 inches. In some embodiments, the pitch may be 0.004-0.007 inches. The larger the pitch is, the easier for glue or the like to reach between the inner piece 124 and the core shaft 110 to more strongly attach the inner piece 124 or the outer piece 125 to the core shaft 110 while not compromising the compliance of the delivery guide wire.

Referring to Figs. 8 and 9, in a further embodiment of the present invention, the inner piece 124 is a meshed tubular structure braided from a wire. In this case, openings in the meshed tubular structure provide the interspace 126.

In this embodiment, one or more of the above-described driving members 120 may be provided on the core shaft 110. In each driving member 120, the inner piece 124 may be braided from a wire that is as thin as applicable. For example, the wire may have a diameter (or cross-sectional width) of 0.001 inch or less. Additionally, the wire may be so braided that there is only a small number, preferably 15-50, braid knots per inch of the inner piece 124. In this way, the driving members 120 may be firmly attached to the core shaft 110 while not compromising the compliance of the delivery guide wire 100.

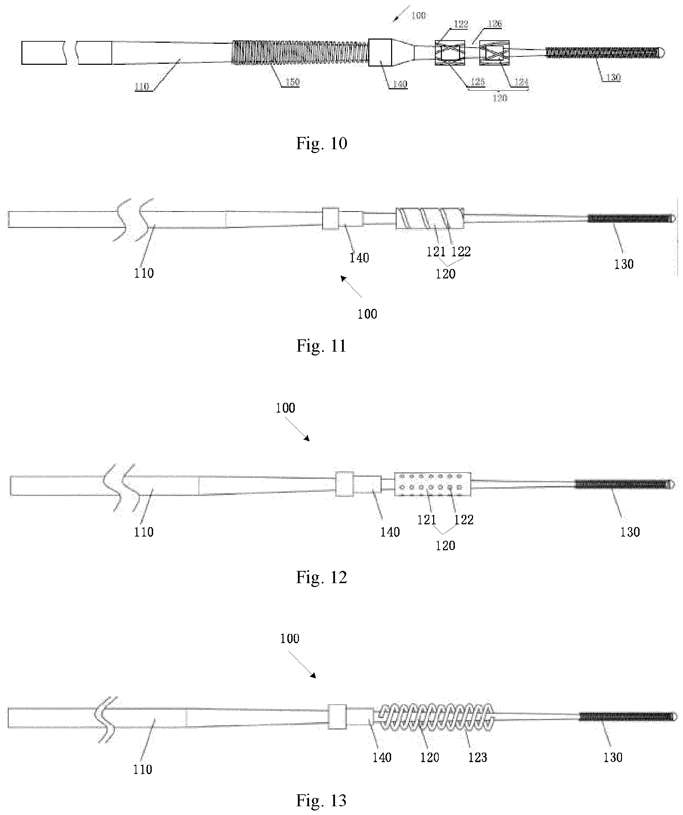

Fig. 10 is a schematic illustration of a further embodiment of the present invention. As shown in Fig. 10, one or more of the above-described inner pieces 124, each implemented as a metal tube, may be welded onto the core shaft 110. In this embodiment, each metal tube may be a tubular member with a neat, smooth surface. A length of each metal tube may be 0.3-2 mm in order to not adversely affect the compliance of the delivery guide wire 100. When at least two such metal tubes are provided, the outer piece 125 may be designed to entirely wrap all the metal tubes, as well as bare surface portion(s) of the core shaft 110 between the metal tubes (which make(s) up the interspace 126), while further extending over the core shaft 110. Alternatively, each metal tube, i.e., each inner piece 124, may be wrapped by a separate outer piece 125 (see Fig. 9). Stated in another way, the individual inner pieces 124 are wrapped with respective outer pieces 125. In addition, each of the outer pieces 125 may wrap portion(s) of the core shaft 110 between the metal tubes (which provide(s) the interspace 126). The wrapping of the inner pieces 124 by the outer pieces 125 may be accomplished by dipping or hot pressing.

In alternative embodiments, each metal tube may have pits, slots or through holes formed, for example, by laser etching. This can further enlarge the interspace 126 and result in an even larger contact area and enhanced attachment strength between the outer piece 125 and the inner piece 124.

In the foregoing embodiment, the inner piece 124 may be formed of a radiographically invisible or visible metal. Examples of the radiographically invisible metal may include, but are not limited to, stainless steel. Examples of the radiographically visible metal may include, but are not limited to, a platinum-tungsten or platinum-iridium alloy. Preferably, the inner piece 124 is formed of a radiographically visible (or radiopaque) metal so that the driving member 120 is visible in a radiographic manner. More specifically, the inner piece may be formed of a material selected from one or more of platinum, gold, tungsten, a platinum-gold alloy, a platinum-tungsten alloy, a platinum-iridium alloy and a platinum-nickel alloy. For example, it may be formed of either or both of a platinum-tungsten alloy and a platinum-iridium alloy (in the latter case, for example, it may be a meshed tubular structure braided both from wires of the platinum-tungsten alloy and from wires of the platinum-iridium alloy). Advantageously, this allows for the operator to accurately determine whether the stent being delivered is retrievable or not. In particular, in practice, the delivery catheter in which the stent is crimped on the delivery guide wire may have a first proximal end and a first distal end opposing the first proximal end, and a visualization ring (not shown) may be provided at the first distal end. During delivery, upon the driving member 120 coming into coincidence with the visualization ring, as viewed in a radiographic image, the operator may know that the stent cannot be retrieved anymore if it is further advanced distally. Therefore, the radiographic visibility of the driving member 120 allows accurate location with the aid of the radiographic imaging device, which greatly facilitates the operator's operation.

In the embodiments shown in Figs. 5 to 10, in each driving member 120, the metallic inner piece 124 is fixedly attached over the core shaft 110, and the polymeric outer piece 125 is fixedly attached over the inner piece 124. In this way, attachment of the outer piece 125 to the core shaft 110 is indirectly enhanced by the inner piece 124, minimizing the risk of loosening, wrinkling or displacement of the driving member 120 during delivery of the stent and increasing safety and reliability of the delivery guide wire 100 during its use for delivering the medical implant. Moreover, when crimped, the medical implant is at least partially received in the recess 122 in the outer surface of the outer piece 125 in the driving member 120, resulting in an enlarged contact area between the stent and the driving member 120 and hence greater friction between the medical implant and the delivery guide wire 100, as well as in increased neatness and smoothness of a portion of the medical implant where it is brought into contact with the delivery catheter and hence reduced friction between the medical implant and the delivery catheter and easier delivery of the medical implant.

In some embodiments, referring to Fig. 11, the recess 122 formed on the body 121 may be a continuous helical groove extending helically in the outer surface of the body 121 about the axis thereof. In these embodiments, the recess 122 is preferably formed by patterning using a wire.

Alternatively, referring to Fig. 12, the recess 122 may include at least two recessed elements spaced across the body 121, which may be circular, square, rhombic or otherwise in shape (i.e., these recessed elements are pits). Moreover, when more than two recessed elements are included, they may be scattered across the outer surface of the body 121 as required. In this embodiment, the recess 122 may be engraved or otherwise formed.

Continuing the example of Fig. 4, the stent 300 will be radially thicker at braid knots 311 where different filaments 310 intersect than at the rest of the stent 300. In this case, the stent 300 may be disposed over the delivery guide wire 100 so that the braid knots 311 are partially received in the recessed elements. In alternative embodiments, the stent may be radially thicker at certain points. In these embodiments, the stent may be disposed over the delivery guide wire so that the radially thicker points are partially or entirely received in the recessed elements. In this way, when disposed over the delivery guide wire 100 and inserted in the lumen of the delivery catheter, the filaments or struts in the stent 300 will have an increased contact area with the body 121, resulting in a greater first friction force between the stent 300 and the driving member 120. Moreover, the stent 300 will have a more uniform outer appearance with a neater, smoother surface where the stent 300 comes into contact with the delivery catheter (i.e., the outer surface of the stent 300), resulting in a smaller second friction force between the stent and the inner wall of the delivery catheter, which reduces the resistance during the delivery of the stent.

As shown in Fig. 13, in another embodiment of the present invention, the driving member 120 may consist of a wound wire wound on the core shaft 110. The wound wire may be helically wound multiple turns about the axis of the core shaft 110 to form a helix with gaps 123 present between adjacent turns. In this case, the gaps 123 make up the depression.

In this embodiment, the number of turns and a pitch of the wound wire may vary as required. For example, if the stent to be delivered by the delivery guide wire 100 has a relative large PPI (for a braided stent, PPI measures the number of braid knots per unit length of the braided stent; here, the length refers to an axial size of the stent), the wound wire on the core shaft 110 may have a large number of turns and a smaller pitch in order to allow for the stent to have an increased contact area with the driving member 120.

Optionally, the wound wire may be a polymeric wire preferably having a relatively high surface coefficient of friction. A suitable wound wire may be selected as practically required.

Optionally, the wound wire may consist of a metal wire and a polymeric coating on an outer surface of the metal wire. Preferably, the metal wire may be a radiopaque metal wire such as a platinum-tungsten alloy wire or a platinum-iridium alloy wire. In this case, the driving member 120 may be itself radiographically visible and enable the operator to easily determine, during delivery of the stent, where the stent is currently located and whether the stent that has been partially released can be retrieved back into the delivery catheter. Specifically, the delivery catheter in which the stent is advanced and delivered may have a second distal end and an opposing second proximal end where a third visualization member may be disposed. In this case, during delivery of the stent, upon the driving member 120 coming into coincidence with the third visualization member, as viewed in a radiographic image, the operator may know that the stent cannot be retrieved any longer.

It would be appreciated that, in the embodiments shown in Figs. 11 to 12, the driving member 120 may include the inner piece 124 and the outer piece 125 as illustrated and described with respect to any of Figs. 5 to 9, whose constructions and materials may be the same as the above embodiments and, therefore, need not be described in further detail herein.

Embodiments of the present invention also provide a therapeutic device including a delivery catheter, a medical implant and the delivery guide wire as defined above. The delivery catheter defines a lumen extending therethrough axially, which is configured to receive the medical implant therein in such a manner that the medical implant is compressed against a wall of the lumen and thus tightly crimped on the driving member 120 so as to be at least partially received in the depression to allow an increased first friction force to be created with the driving member 120. Here, the medical implant is, for example, a self-expanding stent, in particular, a braided or cut stent.

It is to be noted that in order for the driving member 120 to better fit with the medical implant to simplify the assembly of the therapeutic device, according to embodiments of the present invention, the delivery guide wire 100 is preferably specially made for the medical implant. In other words, the medical implant to be delivered thereby is provided, and the forms of the driving member 120 and the depression are determined according to the structure of the medical implant, before the delivery guide wire 100 is fabricated.

Further, a radial size of the lumen of the delivery catheter may vary as practically required and preferably range from 0.017 inches to 0.029 inches. More preferably, the radial size of the lumen is 0.027 inches or less, or 0.021 inches or less. Since the depression on the driving member of the delivery guide wire allows the medical implant to have a reduced outer diameter in the crimped configuration, the lumen of the delivery catheter in the therapeutic device of the present invention is allowed to have a reduced radial size, which in turn reduces the outer diameter of the delivery catheter. The thinner delivery catheter can reach a more distant blood vessel or a smaller lesion, resulting in a wider scope of indications and enhanced overall compliance of the therapeutic device. Moreover, it can more easily pass through tortuous blood vessels to successfully reach a target lesion site, resulting in a higher surgical success rate.

Although the present invention has been disclosed as above, it is not limited to the above disclosure in any sense. Various changes and modifications can be made by those skilled in the art to the present invention without departing from the spirit and scope thereof. Accordingly, it is intended that any and all such changes and modifications are also embraced within the scope of the invention as defined in the appended claims and equivalents thereof.

## Claims

1. A delivery guide wire, for delivering a medical implant, wherein the delivery guide wire comprises a core shaft and a driving member on the core shaft, a depression is defined on the driving member.

2. The delivery guide wire according to claim 1, wherein the driving member comprises a body and a recess formed in an outer surface of the body, and the recess forms the depression.

3. The delivery guide wire according to claim 2, wherein the recess has a structure matching at least a part of a structure of the medical implant when the medical implant is in a crimped configuration.

4. The delivery guide wire according to claim 3, wherein the recess is mirrored to an inner surface of the medical implant in the crimped configuration.

5. The delivery guide wire according to claim 3, wherein the recess has a width ranging from 0.0008 inches to 0.004 inches, and/or the recess has a depth ranging from 0.0002 inches to 0.004 inches.

6. The delivery guide wire according to claim 2, wherein the recess comprises one recessed element or a plurality of recessed elements which are interlaced or continuous with, or spaced apart from, one other across the outer surface of the body.

7. The delivery guide wire according to claim 2, wherein the recess helically extends in the outer surface of the body about an axis thereof to form one or more helical grooves.

8. The delivery guide wire according to claim 1, wherein the driving member comprises a wound wire which helically extends about an axis of the core shaft to form one or more helices, and adjacent turns of the wound wire spaced apart to form the depression.

9. The delivery guide wire according to claim 8, wherein the wound wire is a polymeric wire or a metal wire coated on a surface thereof with a polymeric coating.

10. The delivery guide wire according to claim 9, wherein the metal wire is visualizable, and/or the metal wire is a platinum-tungsten alloy wire or a platinum-iridium alloy wire.

11. The delivery guide wire according to claim 1, wherein the driving member comprises an inner piece and an outer piece,
wherein the inner piece is made of a metallic material and fixedly sleeved over the core shaft, and
the outer piece is made of a polymeric material and fixedly sleeved over the inner piece.

12. The delivery guide wire according to claim 11, wherein the inner piece defines an interspace which is partially or entirely filled by the outer piece, and wherein at least a part of the outer piece goes through the interspace to connect to the core shaft.

13. The delivery guide wire according to claim 11, wherein the inner piece has an outer surface provided therein with a recess which forms the interspace.

14. The delivery guide wire according to claim 11, wherein the inner piece comprises a plurality of coils which are arranged along an axis of the core shaft, and the interspace is formed between adjacent ones of the coils.

15. The delivery guide wire according to claim 11, wherein the inner piece is a meshed tubular structure braided from a wire, and openings in the meshed tubular structure form the interspace.

16. The delivery guide wire according to claim 15, wherein the wire has a diameter of 0.001 inch or less, and/or each inch of the inner piece contains 15-50 braid knots.

17. The delivery guide wire according to claim 11, wherein the inner piece comprises at least one tubular element and is partially or entirely wrapped by the outer piece.

18. The delivery guide wire according to claim 11, wherein the inner piece is a helix formed by a wire extending helically about an axis of the core shaft, and the interspace is formed between adjacent turns of the wire.

19. The delivery guide wire according to claim 18, wherein the wire has a diameter of 0.001 inch or less, and/or the helix formed by the wire has a pitch of 0.001-0.007 inches.

20. The delivery guide wire according to any one of claims 11 to 19, wherein the inner piece is made of a visualizable metallic material selected from one or more of platinum, gold, tungsten, a platinum-gold alloy, a platinum-tungsten alloy, a platinum-iridium alloy and a platinum-nickel alloy.

21. The delivery guide wire according to any one of claims 11 to 19, wherein the inner piece is welded or glued to the core shaft, and/or the outer piece wraps the inner piece and extends to connect to the core shaft.

22. The delivery guide wire according to any one of claims 11 to 19, wherein the outer piece is made of a material comprising one or more of a block polyether amide resin, a thermoplastic polyurethane elastomer, silicone, nylon and an acrylic polymer.

23. The delivery guide wire according to any one of claims 11 to 19, wherein the outer piece is formed on the inner piece by hot pressing and/or dipping, or the inner piece is adhesively bonded to the outer piece.

24. The delivery guide wire according to claim 23, wherein the recess is formed in an outer surface of the outer piece, and/or the recess has a structure matches at least a part of a structure of the medical implant when the medical implant is in a crimped configuration.

25. The delivery guide wire according to claim 24, wherein the recess comprises one recessed element or a plurality of recessed elements which are interlaced or continuous with, or spaced apart from, one other across an outer surface of the body.

26. The delivery guide wire according to any one of claims 1 to 19, wherein at least two of the driving members are provided on the core shaft and are spaced apart along an axis of the core shaft.

27. The delivery guide wire according to claim 26, wherein adjacent ones of the driving members are spaced apart by a distance ranging from 0.5 mm to 150 mm.

28. The delivery guide wire according to claim 27, wherein adjacent ones of the driving members are spaced apart by a distance ranging from 0.5 mm to 5 mm.

29. The delivery guide wire according to any one of claims 1 to 19, wherein one or more of the driving members are provided on the core shaft, and each of the driving members has an outer diameter of 0.01-0.03 inches and a length of 0.5-8 mm.

30. The delivery guide wire according to claim 29, wherein the length of each of the driving member ranges from 0.5 mm to 4 mm.

31. The delivery guide wire according to any one of claims 1 to 19, further comprising a first visualization member and a second visualization member, the first visualization member disposed at a distal end of the core shaft, the second visualization member disposed on the core shaft, wherein the driving member is situated between the first visualization member and the second visualization member.

32. A therapeutic device, comprising a delivery catheter, a medical implant and the delivery guide wire according to any one of claims 1 to 29, wherein the delivery catheter defines a lumen extending therethrough axially, the lumen is configured to receive the medical implant therein so that the medical implant is compressed against a wall of the lumen to become a crimped configuration where the medical implant is sleeved over the driving member so as to be at least partially received in the depression.

33. The therapeutic device according to claim 32, wherein the lumen has a radial size ranging from 0.017 inches to 0.029 inches.

34. The therapeutic device according to claim 32, wherein the medical implant is a self-expanding stent.
